# EUROPEAN PATENT APPLICATION

(11) **EP 2 460 542 A1**
(43) Date of publication of application: **06.06.2012**
(21) Application number: 10804449.6
(22) Date of filing: 28.07.2010
(51) Int. Cl.: A61K 51/00, A61K 38/22, G01N 33/53, G01N 33/531, G01N 33/534

(54) **PANCREATIC ENDOCRINE CELL INDICATOR AND UTILIZATION OF SAME**

(30) Priority: 31.07.2009 JP 2009179935
(71) Applicant: Riken, Wako-shi, Saitama 351-0198 (JP)
(72) Inventor: WATANABE, Yasuyoshi, Kobe-shi Hyogo 650-0047 (JP); SAKO, Takeo, Kobe-shi Hyogo 650-0047 (JP); HASEGAWA, Koki, Kobe-shi Hyogo 650-0047 (JP); KANAYAMA, Yousuke, Kobe-shi Hyogo 650-0047 (JP); KATAOKA, Yousuke, Kobe-shi Hyogo 650-0047 (JP)
(74) Representative: Bittner, Thomas L.
(86) International application number: PCT/JP2010/062701
(87) International publication number: WO 2011/013703

(57) **Abstract**

A pancreatic endocrine cell indicating agent of the present invention includes a modified polypeptide in which a metal chelator binds only to an amino acid that is located at a site other than a somatostatin receptor-binding active site in a polypeptide selected from the group consisting of somatostatin and somatostatin analogs; and a radioactive metal nuclide held by the metal chelator. According to the present invention, it is therefore possible to provide a novel pancreatic endocrine cell indicating agent.

## Description

### Technical Field

The present invention relates to a pancreatic endocrine cell indicating agent and utilization of the same.

### Background Art

Diabetic patients are largely classified into patients of Type 1 diabetes and patients of Type 2 diabetes. Conventionally, Type 1 diabetes was thought to be caused by a reduction in the number of pancreatic beta cells, whereas the Type 2 diabetes was thought to be caused by a decline in function of pancreatic beta cells. However, in recent years, it was successively reported, mainly based on examination of autopsy samples, that a reduction in the number of pancreatic beta cells is observed also in Type 2 diabetes (Non-Patent Literatures 1 through 4). Furthermore, it is becoming clear that a reduction in the number of pancreatic beta cells is observed not only in an advanced stage of diabetes, but also before development of overt diabetes (Non-Patent Literatures 1 through 4).

### Citation List

Non-Patent Literature 1
   Bulter AE et. al. , Diabetes 52 , p. 102-110 , 2003
Non-Patent Literature 2
   Yoon KH et. al. , J. Clin. Endocrinol. Metab. 88, p.2300-2308 , 2003
Non-Patent Literature 3
   Donath MY and Halban PA., Diabetologia 47(3), p.581-589, 2004
Non-Patent Literature 4
   Rhodes CJ., Science 307(5708), p.380-384 , 2005
Non-Patent Literature 5
   Kung MP, Hou C, Kung HF et al., J. Nuclear Medicine 49, p.1171-1176, 2008
Non-Patent Literature 6
   Simpson NR, Souza F, Harris P et al., Nuclear Medicine and Biology Vol.33, No. 7, p.855-864, 2006
Non-Patent Literature 7
   Simpson NR, Souza F, Harris P et al., Journal of Clinical Investigation Vol.116, No.6, p. 1506-1513, 2006
Non-Patent Literature 8
   A. Schmitz, C.-Y. Shiue, Q. Feng, et al., Nuclear Medicine and Biology, vol.31, no.4, p.483-491, 2004
Non-Patent Literature 9
   B. Wangler,.S. Schneider, O. Thew, et al., Nuclear Medicine and Biology, vol.31, no.5, p.639-647, 2004
Non-Patent Literature 10
   Tanaka et al.,Angew. Chem. Int. Ed.2008, 47, p.102-105

### Summary of Invention

### Technical Problem

Currently, autopsy or biopsy is widely performed to measure pancreatic beta cell mass (BCM). These measuring methods are highly invasive, and therefore cause a very large burden on the patients. It is therefore difficult to perform BCM measurement by these methods before development of diabetes and to perform BCM measurement repeatedly in order to grasp a quantitative change with time. Meanwhile, development of convenient BCM measurement can easily realize prevention of onset, diagnosis at earlier stages, further investigation of pathogenesis of diabetes, and monitoring the curative effect of therapy for diabetes. In addition, development of convenient BCM measurement, for example, makes it possible to easily quantify the transplanted pancreatic endocrine cells or tissues and to measure the residual endocrine cells after partial removal of the pancreas for cancer treatment.

Accordingly, development of a less invasive BCM measurement method as compared with autopsy and biopsy is required. A representative example of less invasive methods for BCM measurement is using positron emission tomography (PET) and several papers have been reported about PET probe for BCM measurement.

For example, it is reported that [¹⁸F]9-fluoropropyl-(+)-Dihydrotetrabenazine (DTBZ) for VMAT2 (Vesicular Monoamine Transporter 2) can be a PET probe for pancreas observation (Non-Patent Literature 5). Another paper reports that [¹¹C]Dihydrotetrabenazine (DTBZ) for VMAT2 can be a PET probe for observation of pancreatic beta cells (Non-Patent Literatures 6 and 7).

Furthermore, there is a paper about synthesis of ¹⁸F-labeled analog of Glyburide (generic name), a therapeutic agent for hypoglycemia caused by type 2 diabetes, for observation of pancreatic beta cells (Non-Patent Literature 8). Furthermore, another paper reports about synthesis of ¹⁸F-labeled derivative of Repaglinide (generic name), a therapeutic agent for diabetes, for observation of pancreatic beta cells (Non-Patent Literature 9). Note that both of Glyburide and Repaglinide are ligands of sulfonylurea-receptor and have a common mechanism of action.

However, these PET probes have little practical use since they have (i) a problem that DTBZ has not been approved as a drug in Japan, and practical application is therefore difficult (Non-Patent Literatures 5 through 7) and (ii) a problem that an uptake of the PET probe into pancreas is relatively low (Non-Patent Literatures 8 and 9).

Meanwhile, there is a technique of synthesizing a compound in which DOTA (1,4,7,10-tetraazacyclododecane-N,N',N",N"'-tetraacetic acid) is combined with an amino-acid residue of somatostatin via a predetermined linker by using 6π azaelectrocyclization although this technique is not aimed for BCM measurement. The paper of this technique reports that PET study with the above-mentioned compound conjugated ⁶⁸Ga (radioactive metal nuclide) to its DOTA was conducted and the accumulation of the PET probe was seen in the pancreas and other organs of a rabbit (Non-Patent Literature 10).

Non-Patent Literature 10 reports that the DOTA was introduced at the residue of the N terminal side out of two lysine residues of somatostatin so that it might not affect the activity as somatostatin. However, there is no direct proof of this report. The 6π azaelecrocyclization is a reaction that can occur between any amino group and DOTA. Accordingly, it is highly likely that the DOTA is combined with the active site (the other lysine residue) of somatostatin in the PET compound and a capacity of binding to a somatostatin receptor declines. Furthermore, the PET compound mentioned in Non-Patent Literature 10 can be a mixture of plural types of compounds which are different in position of DOTA conjugated to somatostatin, and therefore may cause a problem in quality stability as a PET diagnostic agent. Furthermore, since a molecular weight of the linker in the PET compound is relatively large, it is impossible to rule out the possibility that the linker causes an adverse effect on (i) uptake of the compound by the pancreatic beta cells, (ii) binding between the compound and somatostatin, or (iii) the like.

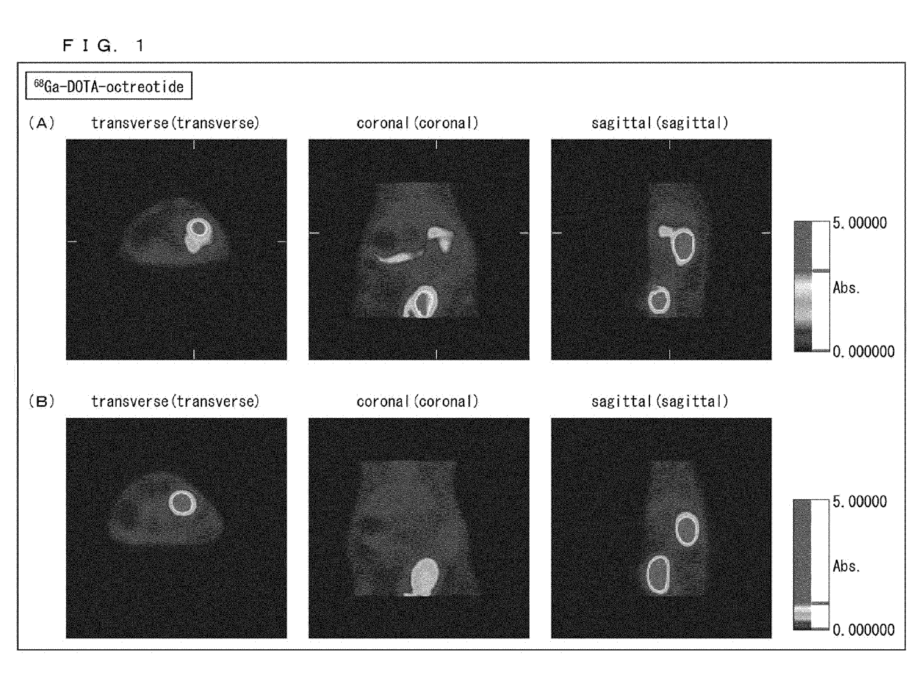

Furthermore, in Non-Patent Literature 10, it has not been confirmed whether the PET compound is accumulated specifically or nonspecifically in pancreas (i.e., whether the PET compound is accumulated due to binding to the somatostatin receptor expressed in the pancreas or just temporarily accumulated in the pancreas in a metabolic process of the PET compound). Further, Fig. 1 etc. of Non-Patent Literature 10 show that the PET compound is highly accumulated in the kidney and the liver as well as the pancreas.

As described in Non-Patent Literature 10, the PET imaging utilizing somatostatin is generally intended to observe a tumor. Therefore, Non-Patent Literature 10 does not motivate a person skilled in the art at all to use the compound for BCM measurement.

The present invention was attained in view of the above problems, and an object of the present invention is to provide a novel pancreatic endocrine cell indicating agent that is more excellent for observation of pancreatic endocrine cells, and utilization of the same.

### Solution to Problem

In order to solve the above problems, the inventors of the present invention conducted diligent studies. As a result, the inventors of the present invention found that a pancreatic endocrine cell indicating agent of the present invention binds specifically to a somatostatin receptor expressed in a pancreatic endocrine cell and most of accumulation of the pancreatic endocrine cell indicating agent in organs other than the pancreas is nonspecific accumulation. Based on this finding, the inventors of the present invention attained the present invention.

That is, a pancreatic endocrine cell indicating agent of the present invention includes: a modified polypeptide in which a metal chelator binds only to an amino acid that is located at a site other than a somatostatin receptor-binding active site in a polypeptide selected from the group consisting of somatostatin and somatostatin analogs; and a radioactive metal nuclide held by the metal chelator.

Further, a pancreatic endocrine cell indicating agent of the present invention includes: a modified polypeptide in which DOTA directly binds only to an amino acid that is located on an amino terminal of a polypeptide selected from the group consisting of somatostatin and somatostatin analogs; and a radioactive metal nuclide held by the DOTA.

According to any of the arrangements, it is possible to provide a novel pancreatic endocrine cell indicating agent which binds specifically to a somatostatin receptor expressed in a pancreatic endocrine cell so that a pancreatic endocrine cell is indicated by the radioactive metal nuclide.

The present invention further provides a diagnostic agent for diabetes that includes any of the pancreatic endocrine cell indicating agents, and a method for measuring pancreatic endocrine cell mass by using any of the pancreatic endocrine cell indicating agents. The measuring method as described above also can be the one to collect the data that help doctors diagnose diabetes.

### Advantageous Effects of Invention

The present invention produces an effect that it is possible to provide a novel pancreatic endocrine cell indicating agent which binds specifically to a somatostatin receptor expressed in a pancreatic endocrine cell so that a pancreatic endocrine cell is indicated by the radioactive metal nuclide.

### Brief Description of Drawings

Fig. 1
   Fig. 1 is a figure of PET images showing how ⁶⁸Ga-DOTA-octreotide, an example of a pancreatic endocrine cell indicating agent of the present invention, binds specifically to a somatostatin receptor expressed in a pancreatic endocrine cell.
Fig. 2

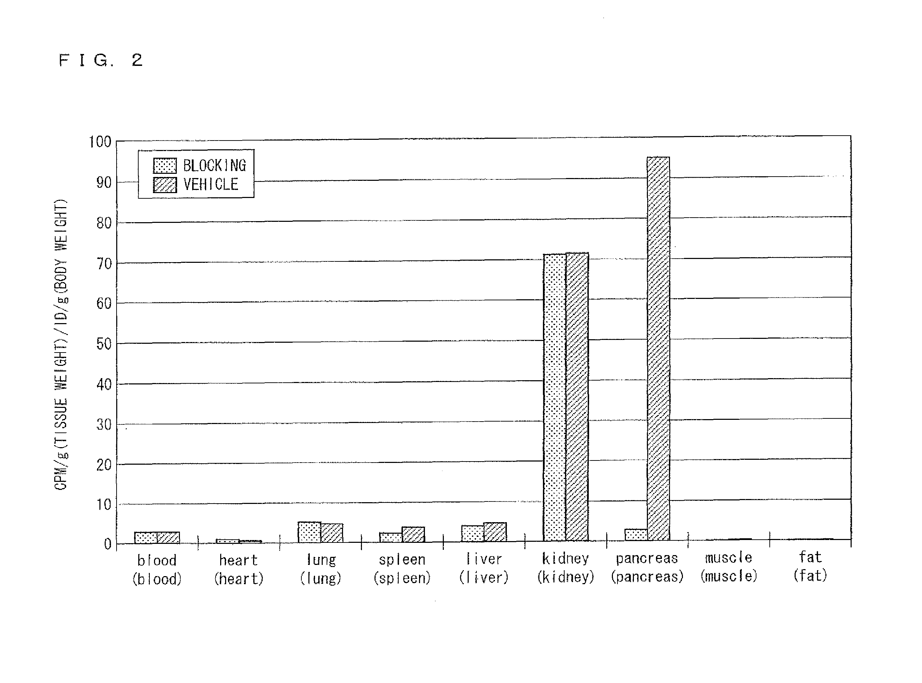

   Fig. 2 is a graph showing a result of quantification of the amount of ⁶⁸Ga-DOTA-octreotide present in organs etc.
Fig. 3

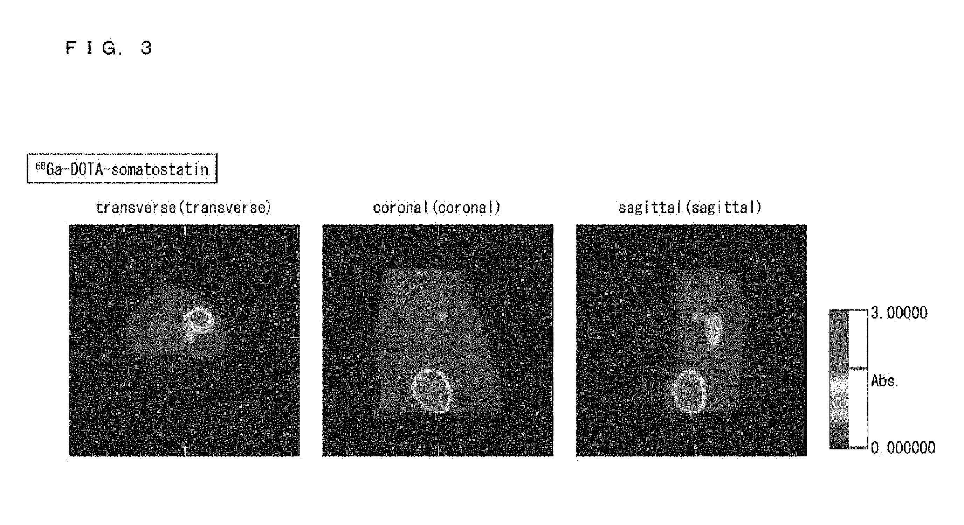

   Fig. 3 is a figure of PET images showing how ⁶⁸Ga-DOTA-somatostatin, an example of the pancreatic endocrine cell indicating agent of the present invention, binds specifically to a somatostatin receptor expressed in a pancreatic endocrine cell.
Fig. 4

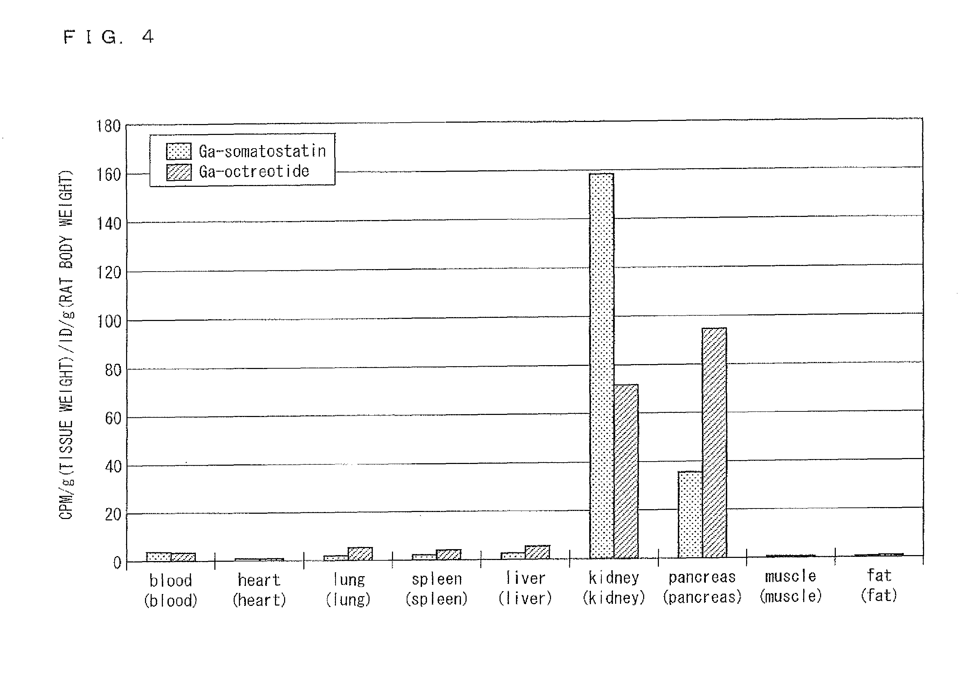

   Fig. 4 is a graph showing a result of quantification of the amount of ⁶⁸Ga-DOTA-octreotide and ⁶⁸Ga-DOTA-somatostatin present in organs etc.
Fig. 5

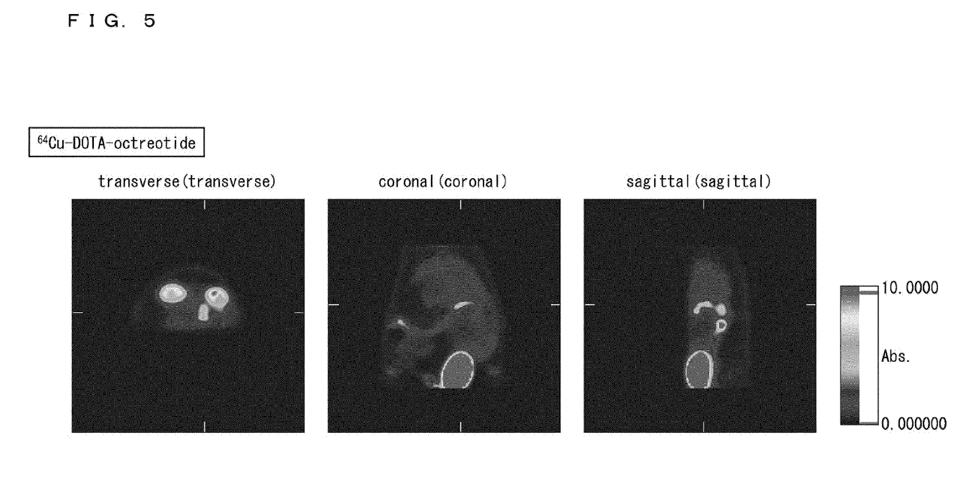

   Fig. 5 is a figure of PET images showing how ⁶⁴Cu-DOTA-octreotide, an example of the pancreatic endocrine cell indicating agent of the present invention, binds specifically to a somatostatin receptor expressed in a pancreatic endocrine cell.
Fig. 6

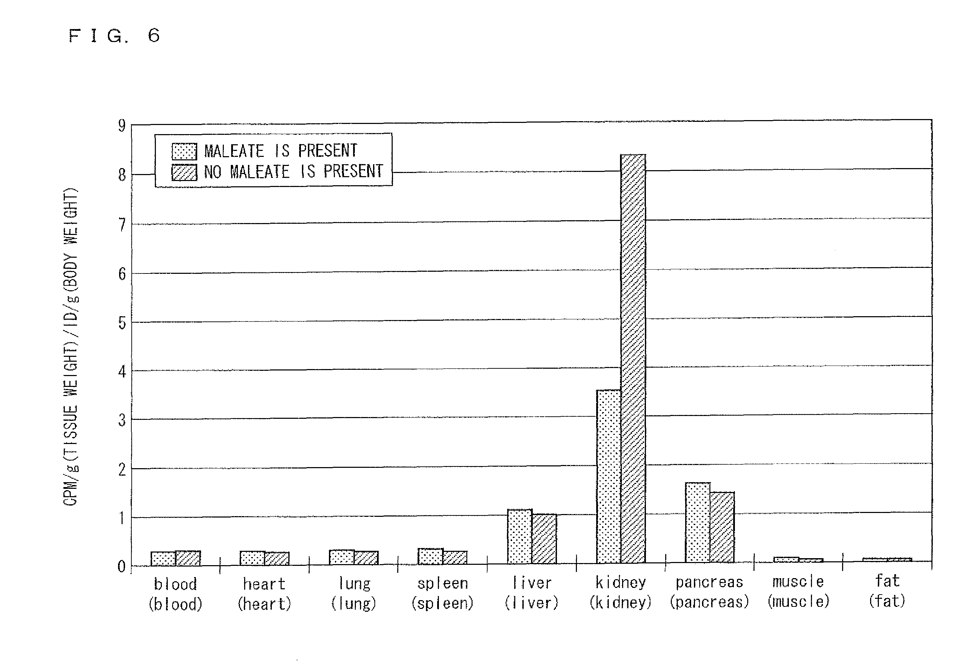

   Fig. 6 is a graph showing a result of quantification of the amount of ⁶⁴Cu-DOTA-octreotide present in organs etc.
Fig. 7

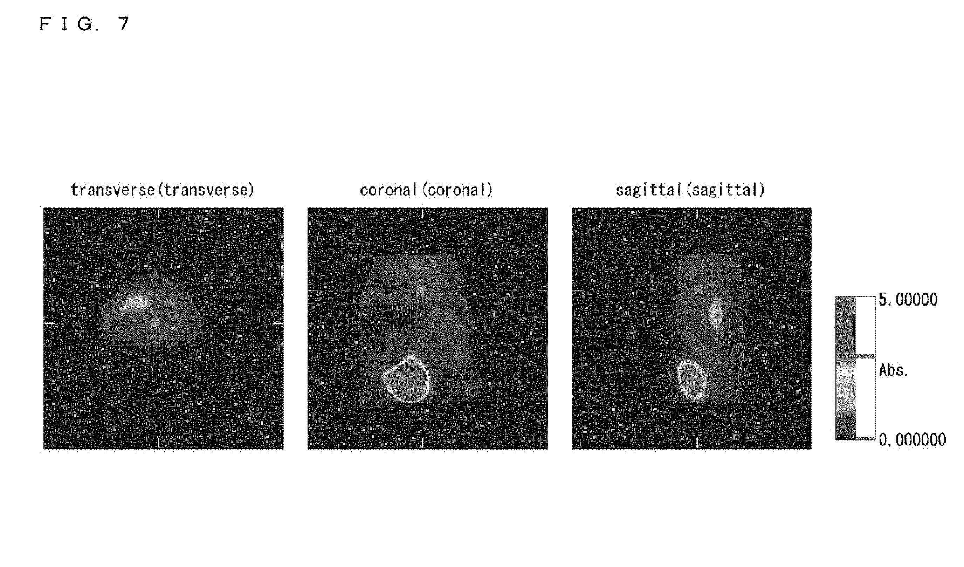

   Fig. 7 is a figure of PET images showing how ⁶⁸Ga-DOTA-octreotide binds specifically to a somatostatin receptor expressed in a pancreatic endocrine cell in a diabetic model rat.
Fig. 8

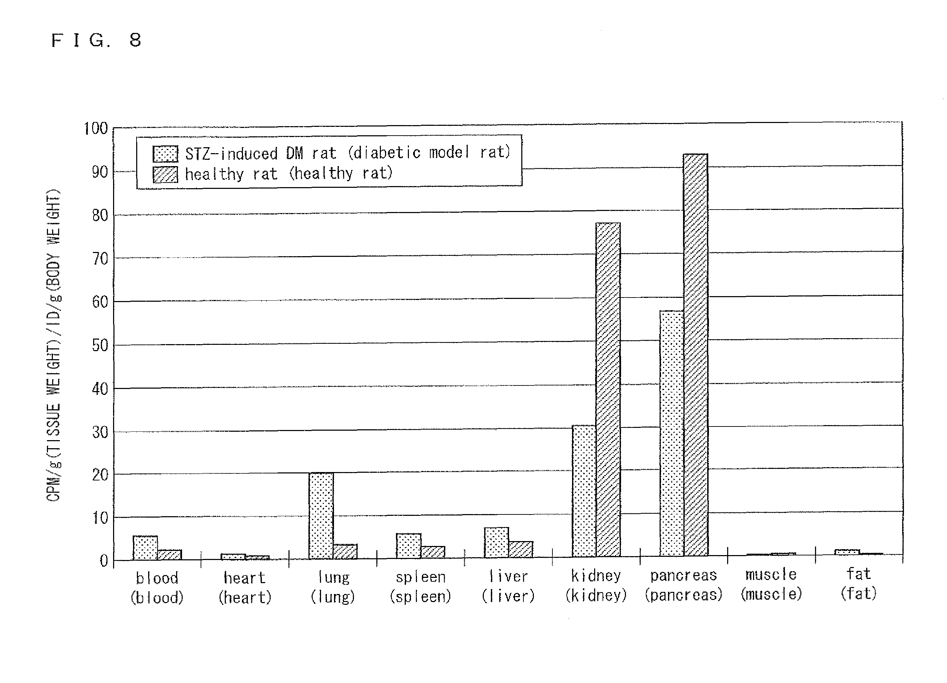

   Fig. 8 is a graph showing a result of quantification of the amount of ⁶⁸Ga-DOTA-octreotide (radioactivity) present in organs etc. of the diabetic model rat.

### Description of Embodiments

An embodiment of the present invention is described below in detail.

### (Pancreatic Endocrine Cell Indicating Agent)

In the present invention, "pancreatic endocrine cell indicating agent (pancreatic endocrine cell measuring agent)" at least contains (i) a modified polypeptide in which a metal chelator binds only to an amino acid that is located at a site other than a somatostatin receptor-binding active site in a polypeptide selected from the group consisting of somatostatin and somatostatin analogs, and (ii) a radioactive metal nuclide held by the metal chelator.

In the present invention, an especially preferable pancreatic endocrine cell indicating agent at least contains (i) a modified polypeptide in which DOTA (1,4,7,10-tetraazacyclododecane-N,N',N",N"'-tetraacetic acid) directly binds only to an amino acid that is located on an amino terminal of a polypeptide selected from the group consisting of somatostatin and somatostatin analogs, and (ii) a radioactive metal nuclide held by the DOTA. This indicating agent binds specifically to a somatostatin receptor that is expressed in a pancreatic alpha cell, a pancreatic beta cell, a pancreatic delta cell, and a pancreatic PP (Pancreatic Polypeptide) cell, each of which is a pancreatic endocrine cell. This indicating agent is a PET probe for indicating presence of an endocrine cell by detecting a gamma ray that occurs by collision between (i) a positron emitted by the radioactive metal nuclide of the indicating agent and (ii) an electron that is present in the vicinity of the radioactive metal nuclide.

In the present invention, what is meant by "somatostatin analog" is a compound which has a capacity of binding specifically to a somatostatin receptor, but has a chemical structure different from somatostatin. Although it is known that somatostatin receptors are classified into 5 sub-types (referred to as SSTR1 through 5, respectively), it is only necessary that the analog should have a capacity of binding specifically to at least one of the 5 sub-types. Examples of the above-mentioned somatostatin analogs include octreotide and octreotide analogs. Examples of the octreotide analogs include [Tyr³]-octreotide, vapreotide, lanreotide, [Tyr³]-octreotate, [Tyr³,Tyr⁸]-octreotide, and the like (see Reference Literature (1) Storch et al., The Journal of Nuclear Medicine, Vol. 46, No. 9, 2005, and Reference Literature (2) Reubi et al., European Journal of Nuclear Medicine, Vol. 27, No. 3, 2000). The concept of the analog also encompasses a compound in which somatostatin or somatostatin analog is subjected to predetermined chemical modification (e.g., polyethylene glycol (PEG) modification). Out of the above examples, octreotide and octreotide analogs are especially preferable from the viewpoints of a longer physiologic half-life (i.e., relatively slow metabolism in or discharge from a living body) and availability for relatively longer observation.

In the present invention, what is meant by "metal chelator (metal chelating agent)" is any chemical structure which can be introduced into somatostatin or somatostatin analogs and which can form a complex with (chelate) metal and hold the metal. Examples of the metal chelator include DOTA, DTPA (diethylene-triamine-pentaacetic acid), HYNIC (6-hydrazinopyridine-3-carboxylic acid), and the like (see Reference Literature: Storch et al., The Journal of Nuclear Medicine, Vol. 46, No. 9, 2005). Each of these metal chelators has a carboxyl group, and can "directly bind" to somatostatin or a somatostatin analog due to a condensation reaction between the carboxyl group and a functional group (e.g., an amino group or a hydroxyl group) inherent in an amino acid constituting somatostatin or the somatostatin analog.

Alternatively, the metal chelator may be one that indirectly binds (referred to as "indirect binding") to somatostatin or a somatostatin analog via a linker. A kind of the linker that can be used is not limited in particular. Examples of the linker include 8-AOC (8-aminooctanoic acid), 5-ADS (5-amino-3-oxapentyl-succinamic acid), 8-AOS (8-amino-3,6-dioxaoctyl-succinamic acid), AMBA (p-aminobenzoic acid), Gly-AMBA, and Gly-AM2BA (Gly-p-aminomethylbenzoic acid) (see Reference Literature: Garrison et al, Bioconjugate Chem. 2008, 19, 1803-1812).

Depending on a chemical structure of a linker to be used, the linker may affect binding between the pancreatic endocrine cell indicating agent of the present invention and a somatostatin receptor. Accordingly, it may be more preferable that the metal chelator directly binds to somatostatin or a somatostatin analog without the use of a linker.

In the present invention, the metal chelator must be introduced directly or indirectly into a site other than a somatostatin receptor-binding active site in somatostatin or a somatostatin analog. This is because the binding activity with the somatostatin receptor greatly declines in a case where the metal chelator is introduced into the binding active site.

Note that the somatostatin receptor-binding active site in somatostatin or somatostatin analogs is known to a person skilled in the art. For example, in the case of somatostatin, the seventh through tenth amino acid sequences (Phe-Trp-Lys-Thr) from the C-terminal side are the binding active site. In the case of octreotide and octreotide analogs, the third through sixth amino acid sequences (sequences corresponding to Phe-Trp-Lys-Thr above) from the C-terminal side are the binding active site. In the present invention, the metal chelator is introduced into a site other than the binding active site. The metal chelator is preferably introduced into an amino acid that is located, on a C-(carboxyl) terminal side or an N-(animo) terminal side, away from the binding active site. For example, the metal chelator is introduced into an amino acid located on the N-terminal side, from the viewpoint of easy introduction (presence of an amino group into which the metal chelator can be introduced).

In the present invention, the expression "DOTA directly binds to an amino acid located on an amino terminal" refers to a state in which DOTA has been introduced due to condensation reaction between a functional group (e.g., an amino group or a hydroxyl group) inherent in an amino acid located on an N-terminal of somatostatin or a somatostatin analog and a carboxyl group of DOTA as described above.

In the present invention, examples of "radioactive metal nuclide" include ⁶⁸Ga, ⁶⁴Cu, and the like, but, in consideration of use as a PET probe, ⁶⁴Cu is especially preferable from the viewpoint of a longer half-life. More specifically, ⁶⁴Cu has a long half-life of approximately 12.7 hours, and therefore has the following practical advantages: (i) capable of observing accumulation of the PET probe at different point in time from the case of using ⁶⁸Ga, (ii) capable of allowing administration to many targets (subjects) by one synthesis, (iii) capable of being used even after being transferred to a remote place, etc.

A formulation in which the pancreatic endocrine cell indicating agent of the present invention is prepared is not limited in particular. The pancreatic endocrine cell indicating agent of the present invention is generally prepared in a liquid form or a suspension form. The liquid or the suspension may contain physiologic saline solution, buffer solution, or the like.

The pancreatic endocrine cell indicating agent of the present invention is used for animals, preferably for mammals, especially preferably for humans. Examples of mammals other than humans include rodents such as mouse, rat, and rabbit; and primates such as marmoset, crab-eating macaque, and chimpanzee. In addition to these animals, the pancreatic endocrine cell indicating agent of the present invention is applicable to pets such as dog and cat.

The pancreatic endocrine cell indicating agent of the present invention is preferably administered to a living animal by injecting the pancreatic endocrine cell indicating agent in various ways. However, the present embodiment is not limited to this. Further, an administered amount per weight is appropriately determined by a person skilled in the art according to need.

The pancreatic endocrine cell indicating agent of the present invention may contain an element other than the modified polypeptide and the radioactive metal nuclide according to need. The element that can be added may be integrated with the modified polypeptide and the radioactive metal nuclide so as to form a composition or may be contained as a separate element from the modified polypeptide and the radioactive metal nuclide.

For example, the pancreatic endocrine cell indicating agent of the present invention may contain a radiolabelled compound in addition to the modified polypeptide that holds the radioactive metal nuclide. However, it is preferable, from the viewpoint of excellent quality stability, that the pancreatic endocrine cell indicating agent of the present invention should contain, as a radiolabelled compound, only one kind out of the modified polypeptide that holds the radioactive metal nuclide.

Furthermore, for example, the pancreatic endocrine cell indicating agent of the present invention may contain "an adjusting agent for adjusting the excreted amount of the modified polypeptide (i.e., somatostatin or a somatostatin analog to which a metal chelator (e.g., DOTA) binds to) holding the radioactive metal nuclide into kidney". The expression "adjusting the excreted amount into kidney" means that the amount of the modified polypeptide to be excreted into kidney is increased or decreased as compared with a case where the adjusting agent is not administered to the living body. Presence of the adjusting agent makes it possible to freely adjust how much the pancreatic endocrine cell indicating agent of the present invention is accumulated nonspecifically in the kidney in a case where the pancreatic endocrine cell indicating agent is administered into the living body. Using adjusting agent has effect to indicate a pancreatic endocrine cell much more specifically by the pancreatic endocrine cell indicating agent of the present invention (see also Examples).

The "adjusting agent" can be, for example, "a compound that is positively charged in order to block megalin which is a scavenger receptor for many proteins and peptides". Various kinds of such compound are known in the pharmaceutical field. Specific examples of a compound that can be used as "adjusting agent" include lysine, arginine, maleic acid, maleate (e.g., sodium maleate), and the like, but maleic acid or maleate is more preferable in point of large reduction in renal accumulation. Meanwhile, lysine or arginine is more preferable in point of less harm to living things.

Furthermore, the present invention can provide a kit which includes a combination of the pancreatic endocrine cell indicating agent of the present invention with administering means (e.g., syringe), a manual (which may be stored in an electronic medium), etc. that are necessary for use of the pancreatic endocrine cell indicating agent.

The pancreatic endocrine cell indicating agent of the present invention is arranged so that a metal chelator binds only to an amino acid that is located at a site other than a somatostatin receptor-binding active site in somatostatin or somatostatin analogs. This reduces a risk of causing a substantial influence on a capacity of binding specifically to the somatostatin receptor. Accordingly, the pancreatic endocrine cell indicating agent of the present invention is an excellent indicator in capacity of binding specifically to a somatostatin receptor.

A suitable example of the pancreatic endocrine cell indicating agent of the present invention is arranged so that DOTA is introduced only into an amino acid that is located on an amino terminal of somatostatin or a somatostatin analog, and therefore has no substantial influence on a capacity of binding specifically to a somatostatin receptor. Accordingly, this suitable example is a more excellent indicator in capacity of binding specifically to a somatostatin receptor, as compared with (i) an arrangement in which DOTA is introduced via a linker, (ii) an arrangement in which DOTA is introduced into an amino acid that is located at a position other than an N-terminal of somatostatin or the like, and (iii) the like arrangement.

In organs (especially the kidney) other than the pancreas, the pancreatic endocrine cell indicating agent of the present invention is accumulated mainly because of nonspecific binding. That is, even in a case where accumulation of the pancreatic endocrine cell indicating agent of the present invention is observed in organs other than the pancreas, percentage of specific binding to the somatostatin receptor is relatively low, and percentage of temporal accumulation in a metabolic process is relatively large. Accordingly, the pancreatic endocrine cell indicating agent of the present invention is more suitably used as an indicating agent for observation of a pancreatic endocrine cell by (i) adjusting a timing at which accumulation in a body is observed after administering the pancreatic endocrine cell indicating agent to a subject (⁶⁴Cu or the like, which has a relatively long half-life, is preferably selected in a case where the accumulation is observed after a relatively long time from the administration) or (ii) administering the adjusting agent, such as maleic acid, to the subject after or before the administration of the pancreatic endocrine cell indicating agent or at the same time as the administration of the pancreatic endocrine cell indicating agent (the adjusting agent may be an element of the pancreatic endocrine cell indicating agent as described above). Note that the processes in (i) and (ii) are not essential, and can be carried out according to need.

In a case where the pancreatic endocrine cell indicating agent (pancreatic endocrine cell measuring agent) of the present invention is used as a PET probe (molecular probe for PET), it is possible to achieve far less invasive observation of a pancreatic endocrine cell. One example is a method for measuring pancreatic endocrine cell mass, which includes the steps of: administering the pancreatic endocrine cell indicating agent of the present invention to a living animal; and measuring gamma-ray activity of the pancreatic endocrine cell indicating agent in the living animal after the administering step. Note that the time interval between the administering step and the measuring step can be appropriately designed by a person skilled in the art according to need, but the time interval is, for example, in a range from 10 minutes to 90 minutes. In the measuring step, imaging using a positron emission tomography is generally used. Pancreatic endocrine cell mass measured in the measuring step is, for example, used for diagnosis of the prediabetic or for judgment of settlement of the implanted pancreatic endocrine cells (including implanted pancreas and pancreatic islet cells). In addition, the pancreatic endocrine cell mass can be also used to measure the residual pancreatic beta cell mass after partial removal of the pancreas for cancer treatment. Note that the above-mentioned measuring method can be a data collecting method for assisting doctor's diagnosis. In this case, the measuring method does not necessarily include the administering step, and is only required to include the measuring step.

The pancreatic endocrine cell indicating agent of the present invention can be used exclusively as an indicator of pancreatic beta cells (e.g., PET probe for observing pancreatic beta cell mass) since (i) percentage of pancreatic beta cells in pancreatic endocrine cells is extremely high (approximately 60% to 70% in the case of humans) and (ii) a somatostatin receptor is highly expressed in a pancreatic beta cell as compared with the other pancreatic endocrine cells. Furthermore, the pancreatic endocrine cell indicating agent of the present invention can be used as "diagnostic agent for diabetes (agent for diabetes testing)" since a remarkable decline in the number of pancreatic beta cells is observed in Type 1 and Type 2 diabetic patients (each also including patients with no explicit symptom).

Note that the term "diagnostic agent for diabetes" refers to a wide variety of agents for judging whether or not a patient has developed Type 1 diabetes or Type 2 diabetes or judging possibility of developing Type 1 diabetes or Type 2 diabetes (risk of becoming evident), by using, as an indicator, pancreatic endocrine cell mass (mainly pancreatic beta cell mass). In these judgments, in a case where "a decline in pancreatic beta cell mass" is observed, it is determined that the patient has "developed diabetes" or that "there is a possibility" of developing diabetes. A decline in pancreatic beta cell mass can be judged, for example, by (i) grasping a change of pancreatic beta cell mass over time in an animal to be examined or (ii) comparing the pancreatic beta cell mass in the animal to be examined with pancreatic beta cell mass (reference value) in a reference animal equivalent to the animal to be examined (same in species, sex, and age as the animal to be examined).

Use of the "diagnostic agent for diabetes" of the present invention, for example, makes it possible to grasp progression of diabetes before a rise in the level of blood sugar is observed (before the symptom of diabetes becomes evident). This allows prevention of onset of diabetes (for example, by promoting improvement of lifestyle) on the evident of early detection of diabetes, early treatment of diabetes, prevention of progression or increase in severity of the disease, remission of the disease, confirmation of the curative effect and the like.

The novel features of the invention described above will be understood more specifically with reference to Examples described later.

### (Synthesis Example of Pancreatic Endocrine Cell Indicating Agent)

The pancreatic endocrine cell indicating agent of the present invention can be synthesized by an appropriate combination of a known peptide solid-phase synthesis method and a known method of introducing a metal chelator into a peptide. That is, the pancreatic endocrine cell indicating agent of the present invention can be synthesized by solid-phase-synthesizing a peptide in which amino acid residues except an amino acid residue into which a metal chelator is to be introduced are protected by a protective group and introducing the metal chelator into the peptide. For example, the pancreatic endocrine cell indicating agent of the present invention can be synthesized in accordance with the descriptions in "Reference Literature: Tetrahedron Letters 44 (2003) 2393-2396" which shows an experimental example in which DOTA, which is a metal chelator, is introduced into [Tyr³]-octreotide (TOC).

Further, an especially preferable pancreatic endocrine cell indicating agent of the present invention can be produced by a method which includes (i) a first step of preparing a mixture solution in which DOTA having no protective group and dimethylsulfoxide (DMSO) are mixed; (ii) a second step of bringing the mixture solution into contact with somatostatin or a somatostatin analog solid-phase synthesized, on a solid-phase support, by a known peptide solid-phase synthesis method, so as to prepare a modified polypeptide in which DOTA directly binds to an amino acid located on an amino terminal of somatostatin or the somatostatin analog; and (iii) a third step of causing DOTA held by the modified polypeptide to chelate (form a complex with) a radioactive metal nuclide.

Here, what is meant by "DOTA having no protective group" is 1,4,7,10-tetraazacyclododecane-N,N',N",N"'-tetraacetic acid, which is represented by the following formula. Note that DOTA may be synthesized by a known method or may be a commercially available one.

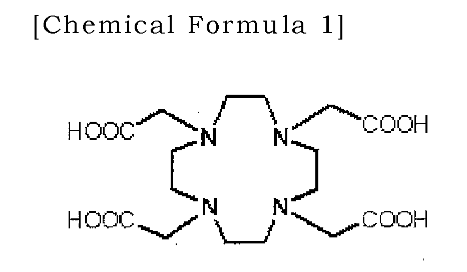

The first step preferably includes a process of activating DOTA with the use of an activating agent. In the present specification, the "process of activating DOTA" can be, for example, a process of reacting DOTA with an appropriate active esterifying component (e.g., N-hydroxyamines such as succinimide, and phenols) so as to form a reactive ester.

In the second step, for example, functional groups of amino acids located at positions other than an amino terminal are protected in a process of synthesizing somatostatin or a somatostatin analog by a known peptide solid-phase synthesis method. This allows DOTA to directly bind only to an amino acid located on the amino terminal of somatostatin or the like. Note that the process of "bringing the mixture solution into contact" in the second step is not limited in particular, provided that it allows a chemical reaction to occur between DOTA and somatostatin or the somatostatin analog into which DOTA is to be introduced.

The process of "causing DOTA to chelate a radioactive metal nuclide" in the third step can be carried out by a known method for causing DOTA to form a chelate with a metal nuclide.

According to the above-mentioned synthesis method, DOTA is introduced only into the amino acid located on the amino terminal of somatostatin or the somatostatin analog. This makes it possible to surely synthesize a "pancreatic endocrine cell indicating agent" which includes a single type of radiolabelled compound. Consequently, no problem occurs in quality stability as an indicating agent and a diagnostic agent.

### (Preferable Arrangement)

The pancreatic endocrine cell indicating agent of the present invention preferably includes, as a radiolabelled compound, only one kind out of the modified polypeptide holding the radioactive metal nuclide, from the viewpoint of excellent quality stability.

The pancreatic endocrine cell indicating agent of the present invention may further include an adjusting agent for adjusting a excreted amount of the modified polypeptide holding the metal nuclide into a kidney.

According to the arrangement, especially an amount of the modified polypeptide to be accumulated in the kidney is adjusted through adjustment of the excreted amount of the modified polypeptide into the kidney. This makes it possible to indicate a pancreatic endocrine cell further more specifically with the use of the pancreatic endocrine cell indicating agent of the present invention. Note that "adjustment of the excreted amount of the modified polypeptide into the kidney" may be an increase or a decrease in excreted amount of the modified polypeptide into the kidney. For example, in a case where the excreted amount of the modified polypeptide into the kidney is increased, an influence of nonspecific accumulation in the kidney can be reduced in a short time by urination.

In any of the above arrangements, the pancreatic endocrine cell indicating agent of the present invention is preferably arranged such that the polypeptide is octreotide.

According to the arrangement, it is possible to provide a pancreatic endocrine cell indicating agent that can be used for observation over a relatively long time.

In any of the above arrangements, from the viewpoint of a longer half-life, the pancreatic endocrine cell indicating agent of the present invention is preferably arranged such that the metal nuclide is ⁶⁴Cu.

### [Examples]

The present invention is described more specifically with reference to Examples etc. below, but is not limited to this.

### [Synthesis Example 1: Synthesis Example of Pancreatic Endocrine Cell Indicating Agent]

### (1) Synthesis of ⁶⁸Ga-DOTA-somatostatin

2 mg of succinimide and 4 mg of 1-ethyl-3-(dimethylaminopropyl)carbodiimido were dissolved into 2 mL of DMSO. To the solution thus obtained, 8 mg of DOTA (metal chelator) was added, and then the solution was stirred at a room temperature for three hours so as to induce activation reaction.

By using, as a starting material, 300 mg (0.52 mmol/g) of Fmoc-Cys(Trt)-(2-C1) trityl resin, somatostatin was synthesized by a standard Fmoc solid-phase synthesis method. Thus, H-Ala-Gly-Cys(Trt)-Lys(Boc)-Asn(Trt)-Phe-Phe-Trp(Boc)-Lys(Boc)-Thr(tBu)-Phe-Thr(tBu)-Ser(tBu)-Cys(Trt)-(2C1)Trt resin was obtained.

To this resin, 2 mL of a suspension obtained by the activation reaction, 2 mL of N-methylpyrrolidone, and 100 µL of di-isopropylamine were added, and these were stirred at a room temperature for 1 hour. This allows DOTA to be introduced into an amino group of alanine located in an amino terminal of somatostatin. This reaction was repeated five times, and it was confirmed, by ninhydrin reaction, that there is almost no amino group that has not been reacted on the solid-phase resin. The resin after the reaction was washed with methanol, and dried under a reduced pressure.

50 mg of the dried resin was treated with a solution containing 10 µL of triisopropylsilane, 25 µL of ethanedithiol, 25 µL of water, and 940 µL of trifluoroacetic acid (TFA) at a room temperature for 2 hours. Thus, deprotection and cleavege from the resin were performed. After the reaction, a crude peptide was precipitated with 5 mL of cold ether, and the precipitation was dissolved into an acetonitrile solution, and purification was performed by reversed-phase HPLC. After the purification, 5% (v/v) DMSO was added to a phosphate buffer solution (pH = 7.4) so as to form a disulfide bond, and purification was performed by reversed-phase HPLC. Note that identification of DOTA-somatostatin was performed by confirming that a molecular weight agrees with a calculated value with the use of a mass spectrometer.

Next, ⁶⁸GaCls was obtained from a ⁶⁸Ge/⁶⁸Ga generator. ⁶⁸GaCl₃ thus obtained was reacted (caused to form a complex) with DOTA-somatostatin in a 1M HEPES buffer solution for 15 minutes under the conditions of 95°C and pH 3.5. Thus, a reaction solution containing ⁶⁸Ga-DOTA-somatostatin was obtained. The reaction solution thus obtained was purified with the use of a C18 solid phase extraction column, and then the purified solution was extracted with the use of ethanol. Thus, a final compound, i.e., ⁶⁸Ga-DOTA-somatostatin which serves as a diagnostic agent was obtained. A specific radioactivity of ⁶⁸Ga-DOTA-somatostatin obtained by this method was approximately 30 GBq/µmol.

### (2) Synthesis of ⁶⁸Ga-DOTA-octreotide

⁶⁸Ga-DOTA-octreotide was synthesized according to the synthesis method of "⁶⁸Ga-DOTA-somatostatin" described above except for that D-Phe-Cys(Trt)-Phe-D-Trp(Boc)-Lys(Boc)-Thr(tBu)-Cys(Trt)-Thr(tBu)-ol-(2C1)Trt resin was obtained by the Fmoc solid-phase synthesis method using, as a starting material, 300 mg (0.20 mmol/g) of Thr(tBu)-ol-(2C1)Trt resin. A specific radioactivity of ⁶⁸Ga-DOTA-octreotide obtained by this method was approximately 30 GBq/µmol.

### (3) Synthesis of ⁶⁴Cu-DOTA-octreotide

DOTA-octreotide in which DOTA was introduced into an amino group of an amino acid located on an amino terminal of octreotide was synthesized according to the synthesis method of "⁶⁸Ga-DOTA-octreotide" described above.

Next, ⁶⁴CuCl₂ was obtained with the use of a medical compact accelerator (product name: CYPRIS HM-12 (produced by Sumitomo Heavy Industries, Ltd.)) by an ordinary method targeting nickel. ⁶⁴CuCl₂ thus obtained was reacted (caused to form a complex) with DOTA-octreotide in a sodium acetate buffer solution for 15 minutes under the conditions of 40°C and pH 5.8 so as to obtain a reaction solution containing ⁶⁴Cu-DOTA-octreotide. The reaction solution thus obtained was purified with the use of a C18 solid phase extraction column, and then the purified solution was extracted with the use of ethanol. Thus, a final compound, i.e., ⁶⁴Cu-DOTA-octreotide which serves as a diagnostic agent was obtained. A specific radioactivity of ⁶⁴Cu-DOTA-octreotide obtained by this method was approximately 70 GBq/µmol.

### [Example 1: PET Imaging of Healthy Rat (1)]

1 nmol (approximately 30 MBq) of ⁶⁸Ga-DOTA-octreotide (pancreatic endocrine cell indicating agent (hereinafter referred to as "PET probe")) obtained in Synthesis Example 1 was dissolved into 500 µl saline, and the solution thus obtained was intravenously administered, under isoflurane anesthesia, to a 8-week-old male Sprague Dawley rat whose blood-sugar level is normal. Next, the abdomen of the rat was scanned with the use of a positron emission tomography (product name: microPET Focus220 (produced by SIEMENS)). The scan was performed for 90 minutes from the administration.

Note that image reconstruction of the scan data was performed as follows with the use of ASIPro (software produced by SIEMENS). Immediately after the administration of the PET probe, the PET probe is in the process of distribution. Accordingly, accumulation of the PET probe observed in an image taken within approximately 10 minutes from the administration is strongly affected by unbound PET probes in blood rather than PET probes binding specifically to a somatostatin receptor. Therefore, scan data obtained during a period of time from 10 minutes later to 90 minutes later from the administration of the PET probe was reconstructed with a Maximum a Posteriori Expectation Maximization algorithm (MAP-EM algorithm: Reference Literature/Qi J, Leahy R, Resolution and noise properties of MAP reconstruction for fully 3-D PET, IEEE Trans Med Imag vol 19, 493-506(2000)).

The image thus reconstructed is shown in (A) of Fig. 1. As shown in (A) of Fig. 1, accumulation of the pancreatic endocrine cell indicating agent (hereinafter referred to as "PET probe") was observed mainly in pancreas, kidney, and liver. Note that, in (A) of Fig. 1, the pancreas is located at an intersection between lines connecting the white lines shown on a periphery of each image.

Further, organs etc. were removed from the rat after the scan, and the PET probe present in blood, heart, lung, spleen, liver, kidney, pancreas, muscle, and fat was quantified. Specifically, radioactivity of the organs etc. thus removed was measured with the use of a gamma counter. The result of the quantification is shown by "VEHICLE" in Fig. 2. The result shown in (A) of Fig. 1 is reflected well in the result of Fig. 2.

### [Example 2: Confirmation of Specificity of PET Probe]

### Blocking study;

The blocking study in the present Example is for examining whether binding of the PET probe is inhibited or not in a case where a large amount of unlabeled compound (1000 times the amount of the PET probe) is administered in advance so that the somatostatin receptor is saturated. In a case where it is revealed, as a result of the experiment, that binding of the PET probe is inhibited, it is determined that the PET probe binds specifically to the somatostatin receptor.

Specifically, 1 mg of (unlabeled) octreotide dissolved into 200 µl saline was administered intravenously, under isoflurane anesthesia, to 7-week-old male Sprague Dawley rat whose blood-sugar level is normal. 1 minute later, 1 nmol (approximately 30 MBq) of ⁶⁸Ga-DOTA-octreotide (PET probe) was dissolved into 300µL saline, and this solution was administered intravenously to the rat under isoflurane anesthesia. Next, the abdomen of the rat was scanned with the use of a positron emission tomography (product name: microPET Focus220 (produced by SIEMENS)). The scan was performed for 90 minutes from the administration. Image reconstruction of scan data was performed in a similar manner to Example 1. The reconstructed image is shown in (B) of Fig. 1. As shown in (B) of Fig. 1, accumulation of the PET probe is observed in kidney and liver, whereas accumulation of the PET probe is remarkably reduced in pancreas.

Further, as in Example 1, organs etc. were removed from the rat after the scan, and the PET probe present in the organs etc. was quantified. The result of the quantification is shown by "BLOCKING" in Fig. 2. The result shown in (B) of Fig. 1 is reflected well in the result shown in Fig. 2. Specifically, accumulation of the PET probe is reduced in the pancreas by approximately 96.6% due to the blocking.

### [Example 3: PET Imaging of Healthy Rat (2)]

⁶⁸Ga-DOTA-somatostatin (pancreatic endocrine cell indicating agent (hereinafter referred to as "PET probe")) obtained in Synthesis Example 1 was administered to 7-week-old male Sprague Dawley rat whose blood-sugar level is normal, as in Example 1. Next, in accordance with the method in Example 1, the abdomen of the model rat was scanned with the use of a PET and image reconstruction was performed. The reconstructed image is shown in Fig. 3. As shown in Fig. 3, accumulation of the PET probe was observed mainly in pancreas, kidney, and liver. Note that, in Fig. 3, the pancreas is located at an intersection between lines connecting the white lines shown on a periphery of each image.

Further, as in Example 1, organs etc. were removed from the rat after the scan, and the PET probe present in the organs etc. was quantified. The result of the quantification is shown by "Ga-somatostatin" in Fig. 4. Further, the result of the PET probe used in Example 1 (i.e., the data shown by "VEHICLE" in Fig. 2) is also shown by "Ga-octreotide" in Fig. 4 for reference.

As shown in Fig. 4, under the measuring conditions in Examples 1 and 2, the pancreatic accumulation of ⁶⁸Ga-DOTA-octoretide was higher than that of ⁶⁸Ga-DOTA-somatostatin, whereas the renal (nonspecific) accumulation of ⁶⁸Ga-DOTA-octreotide was lower than that of ⁶⁸Ga-DOTA-somatostatin. This result proves that there is a case where octreotide is preferable to somatostatin as a polypeptide constituting the pancreatic endocrine cell indicating agent of the present invention.

### [Example 4: PET Imaging of Healthy Rat (3) and Influence of Administration of Maleate]

(1) ⁶⁴Cu-DOTA-octreotide (pancreatic endocrine cell indicating agent (hereinafter referred to as "PET probe")) obtained in Synthesis Example 1 was administered to 7-week-old male Sprague Dawley rat whose blood-sugar level is normal, as in Example 1. Next, in accordance with the method in Example 1, the abdomen of the model rat was scanned with the use of a PET and image reconstruction was performed. Note that the PET scan was performed for 240 minutes after the administration of the PET probe, and data obtained during a period of time from 10 minutes later to 240 minutes later from the administration was used for the image reconstruction. The reconstructed image is shown in Fig. 5. As shown in Fig. 5, accumulation of the PET probe was observed mainly in pancreas, kidney, and liver. Note that, in Fig. 5, the pancreas is located at an intersection between lines connecting the white lines shown on a periphery of each image.

Further, as in Example 1, organs etc. were removed from the rat after the scan, and the PET probe present in the organs etc. was quantified. The result of the quantification is shown by "NO MALEATE IS PRESENT" in Fig. 6.

### (2) Influence of Administration of Maleate

The PET probe was administered to 7-week-old male Sprague Dawley rat whose blood-sugar level is normal in accordance with the method in (1) of the present Example 4 except for that 400 mg of a sodium maleate (maleate /adjusting agent) solution having concentration of 200 mg/mL was added, per kilogram of the rat weight, to 500µL saline in which ⁶⁴Cu-DOTA-octreotide (PET probe) was dissolved. Then, in accordance with the method in (1) of the present Example 4, the abdomen of the rat was scanned with the use of a PET and image reconstruction was performed.

Next, as in the method in (1) of the present Example 4, organs etc. were removed from the rat after the scan, and the PET probe present in the organs etc. was quantified. The result of the quantification is shown by "MALEATE IS PRESENT" in Fig. 6.

As shown in Fig. 6, in a case where maleate was administered, an accumulation amount in the pancreas was slightly increased, whereas an accumulation (nonspecific accumulation) amount in the kidney was slightly reduced. It can be hypothesized that this is mainly because an amount of the PET probe excreted into the kidney was reduced since a blood flow to glomeruli of the kidney was reduced due to a pharmacologic effect of the maleate (effect of contracting blood vessels in the glomeruli).

### [Example 5: Application to Diabetic Model Rat]

### PET Imaging Using Drug-Induced Diabetic Model Rat;

70 mg of Streptozotocin (STZ), which is selectively toxic to the pancreatic beta cells, was intraperitoneally administered to a rat per kilogram of the weight of the rat. Thus, a diabetic rat whose pancreatic beta cells were damaged was prepared. Next, ⁶⁸Ga-DOTA-octreotide (PET probe) was intravenously administered to the model rat in accordance with the method in Example 1. Next, in accordance with the method in Example 1, the abdomen of the model rat was scanned with the use of a PET and image reconstruction was performed. The reconstructed image is shown in Fig. 7. As shown in Fig. 7, accumulation of the PET probe was observed in kidney and liver, whereas accumulation of the PET probe was remarkably reduced in pancreas.

Further, as in Example 1, organs etc. were removed from the model rat after the scan, and radioactivity in each of the organs etc. was quantified. The result of the quantification is shown in Fig. 8.

The present invention is not limited to the description of the embodiments above, but may be altered by a skilled person within the scope of the claims. An embodiment based on a proper combination of technical means disclosed in different embodiments is encompassed in the technical scope of the present invention.

### Industrial Applicability

The present invention is applicable to measurement of pancreatic endocrine cell mass and to diagnosis of diabetes.

## Claims

1. A pancreatic endocrine cell indicating agent comprising:
a modified polypeptide in which a metal chelator binds only to an amino acid that is located at a site other than a somatostatin receptor-binding active site in a polypeptide selected from the group consisting of somatostatin and somatostatin analogs; and
a radioactive metal nuclide held by the metal chelator.

2. A pancreatic endocrine cell indicating agent comprising:
a modified polypeptide in which DOTA (1,4,7,10-tetraazacyclododecane-N,N',N",N"'-tetraacetic acid) directly binds only to an amino acid that is located at an amino terminal of a polypeptide selected from the group consisting of somatostatin and somatostatin analogs; and
a radioactive metal nuclide held by the DOTA.

3. The pancreatic endocrine cell indicating agent according to claim 1 or 2, wherein the pancreatic endocrine cell indicating agent contains, as a radiolabelled compound, the modified polypeptide holding the radioactive metal nuclide and being one selected from the group consisting of variations of the modified polypeptide.

4. The pancreatic endocrine cell indicating agent according to any one of claims 1 through 3, further comprising an adjusting agent for adjusting an excreted amount of the modified polypeptide holding the radioactive metal nuclide into a kidney.

5. The pancreatic endocrine cell indicating agent according to any one of claims 1 through 4, wherein the polypeptide is octreotide.

6. The pancreatic endocrine cell indicating agent according to any one of claims 1 through 5, wherein the radioactive metal nuclide is ⁶⁴Cu.

7. A diagnostic agent for diabetes, comprising a pancreatic endocrine cell indicating agent as set forth in any one of claims 1 through 6.

8. A method for measuring pancreatic endocrine cell mass, comprising the step of measuring gamma-ray activity of a pancreatic endocrine cell indicating agent as set forth in any one of claims 1 through 6 that is present in an animal body.


SEQUENCE LISTING

<110> RIKEN

<120> Indicator for pancreas endocrine cell and use thereof

<130> RK1614PCT

<150> JP2009-179935
<151> 2009-07-31

<160> 2

<170> PatentIn Ver. 2.1

<210> 1
<211> 14
<212> PRT
<213> Artificial Sequence

<220>
<223> Description of Artificial Sequence:Synthesized
      Peptide

<400> 1
Ala Gly Cys Lys Asn Phe Phe Trp Lys Thr Phe Thr Ser Cys
  1               5                  10

<210> 2
<211> 8
<212> PRT
<213> Artificial Sequence

<220>
<223> Description of Artificial Sequence:Synthesized
      Peptide

<400> 2
Phe Cys Phe Trp Lys Thr Cys Thr
  1               5